# EUROPÄISCHE PATENTANMELDUNG

(11) **EP 1 106 148 A1**
(43) Veröffentlichungstag der Anmeldung: **13.06.2001**
(21) Anmeldenummer: 00125770.8
(22) Anmeldetag: 24.11.2000
(51) Int. Cl.: A61F 2/46

(54) **Abziehinstrument zum Lösen einzelner Teile einer Endoprothese**

(30) Priorität: 25.11.1999 DE 19956777; 23.03.2000 DE 10014401
(71) Anmelder: Steinicke Maschinen- und Werkzeugbau AG, 63571 Gelnhausen-Hailer (DE)
(72) Erfinder: Geis, Norbert, 63674 Altenstadt (DE); Hatle, Jaroslav Jan, 63584 Gründau-Lieblos (DE)
(74) Vertreter: Müller, Eckhard, Dr.

(57) **Zusammenfassung**

Es wird eine Vorrichtung (24), insbesondere Abziehinstrument, zum Lösen der einzelnen Teile einer Endoprothese (13), beispielsweise für ein künstliches Hüftgelenk, für Zwischenwirbel-Endoprothesen oder dergleichen, oder zum Spreizen von Wirbelsäulenkörpern, beschrieben. Das Abziehinstrument dient zum Lösen des Preßsitzes von Schaft (3) und Hals (2) oder Hals (2) und Gelenkkugel (1) der Endoprothese (13) und weist zwei Schenkel auf, deren freie Enden (25) in eine Nut (4) zwischen Schaft (3) und Hals (2) oder Hals (2) und Gelenkkugel (1) einführbar sind. Die Schenkel (8)sind mittels einer Spreizvorrichtung voneinander weg bewegbar und treiben die zu lösenden Teile der Endoprothese (13) auseinander (Figur 1).

## Beschreibung

Die Erfindung betrifft eine Vorrichtung, insbesondere Abziehinstrument, zum Lösen einzelner Teile einer Endoprothese, beispielsweise für ein künstliches Hüftgelenk, für Zwischenwirbel-Endoprothesen oder dergleichen, oder zum Spreizen von Wirbelsäulenkörpern, insbesondere zum Lösen des Preßsitzes von Schaft und Hals oder Hals und Gelenkkugel der Endoprothese.

Eine Endoprothese ist u. a. Bestandteil eines künstlichen Hüftgelenks und besteht aus einem Hals, einem Schaft und einer Gelenkkugel. Der Hals ist sowohl in dem Schaft, der in einem Knochen fixiert ist, als auch auf die Gelenkkugel, die in der Gelenkpfanne sitzt, im Preßsitz aufsteckbar. Wenn beim Einsetzen eines künstlichen Hüftgelenks die Endoprothese nicht richtig justiert wird oder auch bei einer Revision der Endoprothese, ist es erforderlich, die Gelenkkugel und/oder gegebenenfalls auch den Schaft wieder vom Hals zu lösen. Bislang kommen dabei Vorrichtungen zum Einsatz, die auf einer Hebelwirkung beruhen. Dabei treten aufgrund der Hebelwirkung unter Umständen sehr große Querkräfte im Oberschenkelbeziehungsweise Beckenbereich auf, wodurch die Prothese in eine Zwangslage gelangen kann, woraus unter Umständen erhebliche Knochenverletzungen resultieren. Darüber hinaus kann die Prothese selbst in Mitleidenschaft gezogen werden, so daß gegebenenfalls neue Prothesenteile eingesetzt werden müssen.

Demgegenüber liegt der Erfindung die Aufgabe zugrunde, eine Vorrichtung der eingangs genannten Art anzugeben, mit der die einzelnen Teile einer Endoprothese in schonender Weise voneinander gelöst werden können und die Probleme bekannter Techniken vermieden werden können.

Diese Aufgabe wird nach der Erfindung bei der Vorrichtung mit den eingangs genannten Merkmalen im wesentlichen dadurch gelöst, daß die Vorrichtung zwei Schenkel aufweist, deren freie Enden in eine Nut zwischen Schaft und Hals oder Hals und Gelenkkugel der Endoprothese einführbar sind und die Schenkel mittels einer Spreizvorrichtung voneinander weg bewegbar sind. Durch diese Maßnahme können durch in die Nut eingeführten Schenkel und Auseinderspreizen der Schenkel die Teile der Endoprothese sicher voneinander gelöst werden. Dabei sind die Schenkel in der Nut sicher gehalten und umfassen bevorzugt den Hals der Endoprothese zumindest über einen Teilbereich. Sobald die Schenkel mittels der Spreizvorrichtung voneinander weg bewegt werden, werden die im Preßsitz miteinander befestigten Teile der Endoprothese schonend und einfach voneinander gelöst, ohne daß große Querkräfte oder dergleichen auftreten könnten.

Die Erfindung läßt sich mit Vorteil auch als Zange zum Auseinanderspreizen benachbarter Wirbelkörper oder für Zwischenwirbelprothesen einsetzen.

Nach einer ersten vorteilhaften Ausgestaltung der Erfindung sind die Schenkel einends mittels eines Bolzenlagers, bevorzugt über Bolzen, an einem Haltegriff verschwenkbar gelagert. Insoweit weist die Vorrichtung in etwa einen zangenähnlichen Aufbau auf, wobei jedoch die beiden Schenkel bevorzugt über jeweils einen zugeordneten Bolzen des Bolzenlagers an dem Haltegriff befestigt sind.

Nach einer anderen vorteilhaften Ausgestaltung der Erfindung sind die freien Endabschnitte der Schenkel an dem den Bolzen abgewandten Abschnitt jeweils als zungenartiges Außenblatt ausgebildet. Hierdurch wird die Voraussetzung geschaffen, daß die zungenartigen Außenblätter in der Ausgangsstellung leicht und einfach in die Nut der Endoprothese eingeführt werden können.

Von besonderem Vorteil sind wenigstens eine, bevorzugt beide der einander zugewandten Innenflächen der Schenkel beziehungsweise Außenblätter im Bereich des freien Endes als Schrägflächen ausgeführt. Die Schrägflächen öffnen sich in Richtung des freien Endes der Außenblätter bevorzugt unter einem zwischen den Schrägflächen eingeschlossenen Winkel von ca. 4°.

Weiterhin hat es sich als vorteilhaft erwiesen, daß mittels der zum freien Ende des Außenblattes geneigten Schrägflächen eine Verjüngung gebildet wird, wobei die Schrägfläche einen Neigungs- beziehungsweise einen Konuswinkel von ca. 2° aufweist beziehungsweise bildet.

Weiterhin hat es sich als äußerst vorteilhaft erwiesen, daß die Spreizvorrichtung ein zwischen Schenkeln beziehungsweise Außenblätter verschiebbar angeordnetes zungenartiges Innenblatt aufweist, welches im Bereich des freien Endes einbevorzugt jedoch zweiseitig komplementäre Schrägflächen besitzt, die jeweils bevorzugt eine Neigung beziehungsweise einen Konuswinkel von ca. 2° aufweisen beziehungsweise bilden.

Dabei weist das Innenblatt aufgrund der Anordnung der Schrägflächen hin zum freien Ende eine Verdickung auf, welche mit der Verjüngung an den freien Enden der Außenblätter korrespondiert.

Die Außenblätter sqwie das Innenblatt besitzen am freien Ende jeweils eine bevorzugt mittig angeordnete, im wesentlichen bogenförmig ausgebildete Ausnehmung, in welcher der Hals der Endoprothese, im wesentlichen bündig dem Rand der Ausnehmung anliegend, aufnehmbar ist. Durch diese Maßnahme wird für einen sicheren Halt der Vorrichtung an den zu lösenden Teilen der Endoprothese gesorgt. In der Ausgangsstellung des Instrumentes steht das Innenblatt gegenüber den Außenblättern um ein gewisses Maß vor beziehungsweise ist das Innenblatt gegenüber den Außenblättern herausgefahren, so daß die halbkreisförmigen Ausnehmungen der Schenkel beziehungsweise der Außenblätter und des Innenblattes im wesentlichen miteinander fluchten.

In dieser Stellung wird das Abziehinstrument mit den Außenblättern und dem Innenblatt in die Nut um Hals der Endoprothese eingeschoben, wobei die Ausnehmungen der Außenblätter und des Innenblattes in die Nut eingreifen. Durch Spreizen der Außenblätter vermittels eines Verschieben des Innenblattes nach innen läßt sich der Hals von der Endoprothese lösen.

Von besonderem Vorteil sind die Außenflächen der Außenblätter in der End- beziehungsweise Lösestellung im wesentlichen parallel zueinander ausgerichtet. In dieser Stellung können die Innenflächen der Außenblätter den Außenflächen des Innenblattes im wesentlichen bündig aufliegen.

Weiterhin weist die Spreizvorrichtung von Vorteil einen Verschiebemechanismus für das Innenblatt auf, mit dem das Innenblatt in die End- beziehungsweise Lösestellung zwischen die freien Enden der Schenkel beziehungsweise Außenblätter einziehbar und auch wieder herausfahrbar ist. Durch diese Maßnahme wird aufgrund der korrespondierend angeordneten Schrägflächen an Außenblättern und Innenblatt durch Einziehen des Innenblattes zwischen die Außenblätter eine Hubbewegung der Außenblätter erzeugt, die die zu lösenden Teile der Endoprothese schonend auseinanderdrückt. Diese Hubbewegung der Außenblätter vollzieht sich im wesentlichen in Richtung der Halsachse der Endoprothese beziehungsweise des Halses, so daß Querkräfte praktisch nicht auftreten.

Von besonderem Vorteil wird der Verschiebemechanismus durch eine Welle mit Gewinde, eine Gewindespindel oder dergleichen gebildet, die drehbar in dem Haltegriff aufgenommen ist und mit einem korrespondierenden Gewinde eines endseitig des Innenblattes angeordneten Schiebeteil in Eingriff steht. Somit kann über eine Drehbewegung dieser Welle beziehungsweise Gewindespindel eine feinfühlige und dosierte Hubbewegung der Außenblätter erzeugt werden.

Nach einer vorteilhaften Ausgestaltung der Erfindung ist die Welle in einem Wellenlager am Haltegriff gelagert, mittels einer Axiallagerung gegen axiales Verschieben gesichert und bevorzugt am freien, aus dem Haltegriff herausragenden Ende mit einem Drehgriff verbunden.

Nach einer weiteren Ausgestaltung der Erfindung besitzt das Schiebeteil einen oberen und/oder einen unteren Längssteg, der oder die in einer korrespondierenden Schlitzung, Ausnehmung oder dergleichen des oder der Schenkel geführt sind. Aufgrund dieser Maßnahme ergibt sich eine sichere Gradführung des Innenblattes während des Verschiebevorganges beziehungsweise Spreizvorganges der Außenblätter.

Von besonderem Vorteil liegt der Hub beziehungsweise die Spreizung der freien Enden der Schenkel beziehungsweise Außenblätter beim Einziehen des Innenblattes zwischen die Außenblätter unter 3%, bevorzugt unter 1%, besonders bevorzugt etwa 0,6 % der Schenkellänge zwischen dem freien Ende und dem Schwenklager des Schenkels.

Nach einem konkreten Ausführungsbeispiel der Erfindung liegt der maximale Hub der Außenblätter unter ca. 1 mm und die Schenkellänge bei etwa 100 mm.

Von besonderem Vorteil bewegt die Spreizvorrichtung die Schenkel beziehungsweise Außenblätter im wesentlichen axial, insbesondere in Richtung der Halsachse der Endoprothese auseinander.

Insgesamt werden bei dem Lösen der Teile der Endoprothese praktisch ausschließlich Kräfte eingesetzt, die in axialer Richtung der Halteachse der Endoprothese wirken. Große Querkräfte, wie sie bei den bekannten Hebeltechniken auftreten, sind nicht vorhanden, die noch verbleibenden, äußerst minimalen Querkräfte werden von der Vorrichtung selbst abgefangen, so daß Schäden an den Einzelteilen der Endoprothese beziehungsweise den Knochen nicht zu befürchten sind. Auch die Hüftgelenkpfanne, in der die Gelenkkugel der Endoprothese einliegt, wird bei einem Lösen der Gelenkkugel vom Hals geschont. Dies ist auf den gezielten Einsatz der praktisch ausschließlich in axialer Richtung wirkenden Lösekräfte zurückzuführen, die über die Hubbewegung der Schenkel beziehungsweise Außenblätter erzeugt werden. Außerdem ist auch durch die äußerst geringe Neigung der Schrägflächen von bevorzugt ca. 2° und den Gewindemechanismus zum Verschieben des Innenblattes relativ zu den Außenblättern für eine äußerst feinfühlige, dosierte Hubbewegung der Außenblätter gesorgt. Auch können durch das enorme Übersetzungsverhältnis zwischen Getriebeeingang am Handrad und Getriebeausgang, nämlich der Hubbewegung der Außenblätter durch relativ geringe Eingangskräfte sehr hohe Kräfte zum Lösen der Teile der Endoprothese im Bereich der Außenblätter erzeugt werden.

Weitere Ziele, Vorteile, Merkmale und Anwendungsmöglichkeiten der vorliegenden Erfindung ergeben sich aus der nachfolgenden Beschreibung eines Ausführungsbeispieles anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigen:
- Figur 1: eine schematische Längsschnittsdarstellung einer Ausführungsform des erfindungsgemäßen Abziehinstrumentes in der Gebrauchsstellung an einer Endoprothese,
- Figur 2: eine Seitenansicht des Abziehinstruments der Figur 1 um 90° um die Längsachse verdreht,
- Figur 3: eine Längsschnittsdarstellung eines Schenkels des Abziehinstrumentes der Figur 1,
- Figur 4: eine Seitenansicht des Schenkels der Figur 3, jedoch um 90° verdreht,

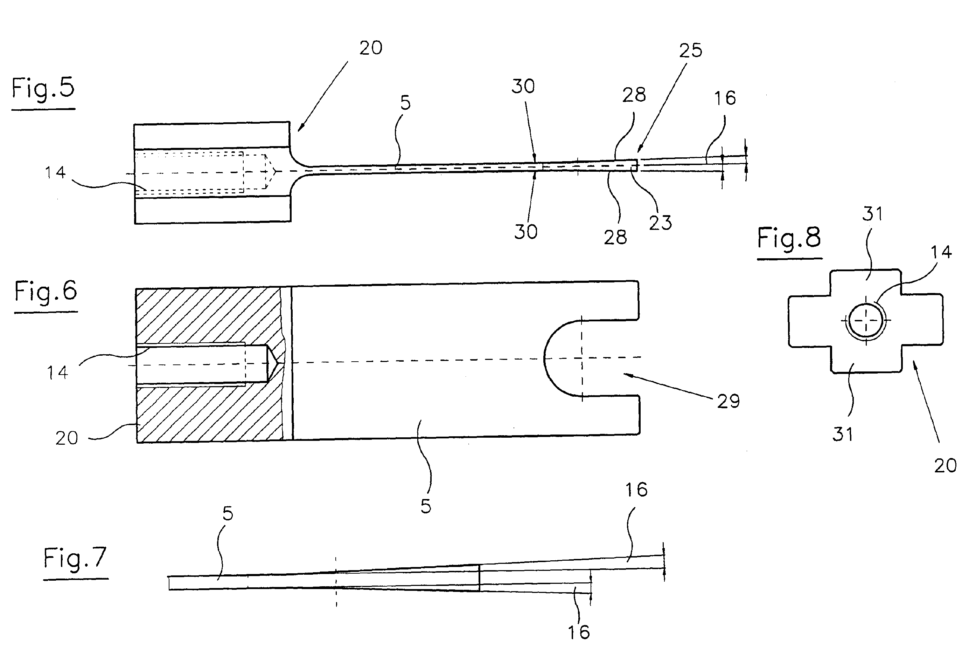

- Figur 5: eine Seitenansicht des Innenblatts mit Schiebeteile des Abziehinstrumentes der Figur 1,
- Figur 6: eine Längsschnittsdarstellung des Innenblatts gemäß Figur 5, jedoch um 90° verdreht,
- Figur 7: eine vergrößerte Darstellung des freien Endes des Innenblattes der Figur 5 und
- Figur 8: eine Rückansicht des Schiebeteils der Figur 5.

Die in den Figuren dargestellte Vorrichtung 24, insbesondere ein Abziehinstrument, dient zum Lösen der einzelnen Teile einer Endoprothese 13, beispielsweise für ein künstliches Hüftgelenk oder dergleichen, insbesondere zum Lösen des Preßsitzes von Schaft 3 und Hals 2 oder Hals 2 und Gelenkkugel 1 der Endoprothese. Hierzu weist die Vorrichtung 24 zwei Schenkel 8 auf, deren freie Enden 25 in eine Nut 4 zwischen Schaft 3 und Hals 2 oder Hals 2 oder Gelenkkugel 1 der Endoprothese 13 einführbar sind. Die Schenkel 8 sind im Bereich der Nut 4 mittels einer Spreizvorrichtung voneinander weg bewegbar. Die Schenkel 8 sind einends mittels eines Bolzenlagers 19, bevorzugt über Bolzen 7, an einem Haltegriff 12 verschwenkbar gelagert. Anderenends im Bereich freier Endabschnitte sind die Schenkel 8 jeweils als zungenartiges Außenblatt 6 ausgebildet, welches beispielsweise eine Dicke von weniger als 2 mm aufweist.

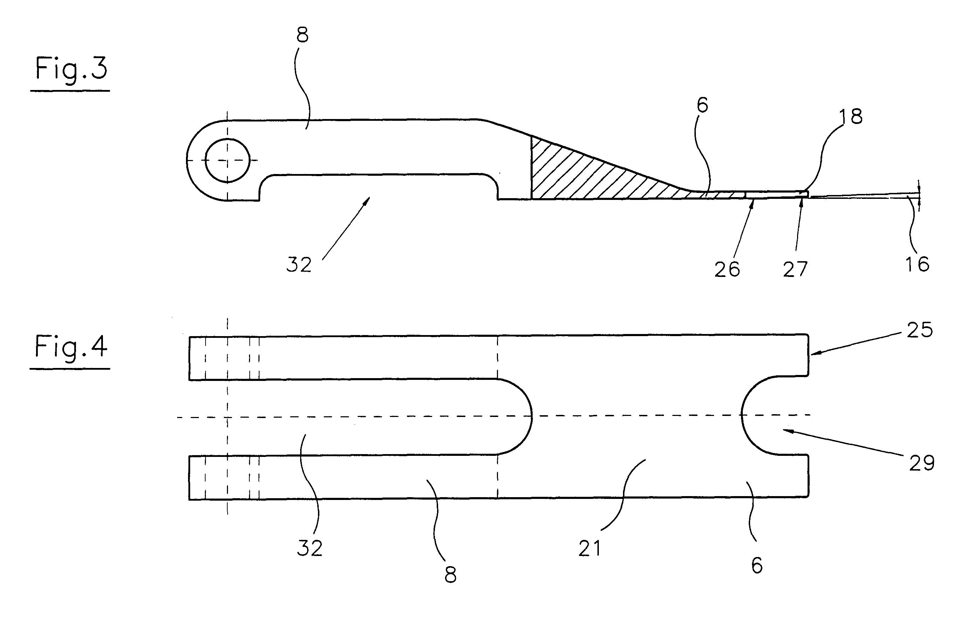

Wie insbesondere aus Figur 3 ersichtlich ist, sind wenigstens eine, bevorzugt beide der aneinander zugewandten Innenflächen 26 der Schenkel 8 beziehungsweise Außenblätter 6 im Bereich des freien Endes als Schrägflächen 27 ausgebildet. Dabei sind die Schrägflächen 27 derart angeordnet, daß an dem freien Ende 25 des Außenblattes 6 eine Verjüngung 18 gebildet wird. Die Neigung der Schrägflächen 27 in Bezug auf eine Mittel-Längs-Ebene beziehungsweise der Konuswinkel der Schrägflächen 27 liegt bei ca. 2°.

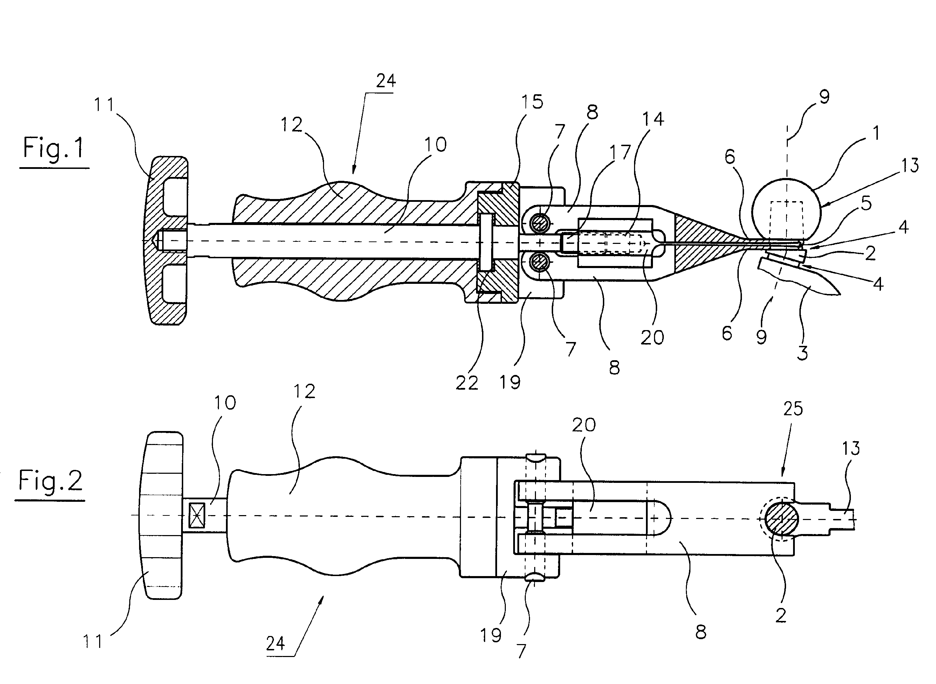

Die Spreizvorrichtung weist ein zwischen den Schenkeln 8 beziehungsweise Außenblätter 6 verschiebbar angeordnetes, zungenartiges Innenblatt 5 auf, welches im Bereich des freien Endes 25 ein- bevorzugt jedoch zweiseitig komplementäre Schrägflächen 28 besitzt, die jeweils bevorzugt eine Neigung von ca. 2° aufweisen beziehungsweise einen Konus 16 von etwa 2° besitzen. Dabei sind die Schrägflächen 28 derart am freien Ende des Innenblattes 5 angeordnet, daß hier eine Verdickung 23 gebildet wird, welche mit den Verjüngungen 18 der Außenblätter 6 korrespondiert. In End- beziehungsweise Löseposition, wie sie in Figur 1 dargestellt ist, liegen bei dem hier gewählten Ausführungsbeispiel die Außenblätter 6 dem Innenblatt 5 beidseitig vollflächig auf, wobei die Außenflächen 21 der Außenblätter 6 in dieser Endbeziehungsweise Lösestellung, in der die Innenflächen 26 der Außenblätter 6 den Außenflächen 30 des Innenblattes bündig aufliegen, im wesentlichen parallel zueinander ausgerichtet sind.

Die Außenblätter sowie auch das Innenblatt besitzen am freien Ende 25 eine bevorzugt mittig angeordnete, im wesentlichen bogenförmig ausgebildete Ausnehmung 29, in die der Hals 2 der Endoprothese 13, im wesentlichen bündig dem Rand der Ausnehmung 13 anliegend, aufnehmbar ist.

Die Spreizvorrichtung weist einen Verschiebemechanismus für das Innenblatt 5 auf, mit dem das Innenblatt 5 in die Endbeziehungsweise Lösestellung zwischen die freien Ende 25 der Schenkel 8 beziehungsweise Außenblätter 6 einziehbar ist. Der Verschiebemechanismus für das Innenblatt 5 ist durch eine Welle 10 mit Gewinde 17, eine Gewindespindel oder dergleichen gebildet, die drehbar in dem Haltegriff 12 aufgenommen ist und mit einem korrespondierenden Gewinde 14 eines endseitig des Innenblattes 5 angeordneten Schiebeteils 20 in Eingriff steht. Die Welle 10 ist des weiteren in einem Wellenlager 15 am Haltegriff 12 gelagert, mittels einer Axiallagerung 22 gegen axiales Verschieben gesichert und bevorzugt am freien, aus dem Haltegriff herausragenden Ende mit einem Drehgriff 11 verbunden.

Das Schiebeteil 20 besitzt einen oberen und unteren Längssteg 31, die in korrespondierenden Schlitzungen, Ausnehmungen 32 der Schenkel 8 längsverschiebbar geführt sind.

Der Hub beziehungsweise die Spreizung der freien Enden der Schenkel 8 beziehungsweise Außenblätter 6 beim Einziehen des Innenblattes 5 zwischen die Außenblätter 6 liegt unter 3%, bevorzugt unter 1%, besonders bevorzugt bei etwa 0,6% der Schenkellänge der Schenkel 8 zwischen dem freien Ende 25 und dem Schwenklager. Nach einem konkreten Ausführungsbeispiel beträgt der maximale Hub der Außenblätter 6 etwa 1 mm oder weniger, wobei die Schenkellänge bei etwa 100 mm liegt.

Die Spreizvorrichtung bewegt die Schenkel 8 beziehungsweise Außenblätter 6 im wesentlichen axial in Richtung der Halsachse 9 der Endoprothese 13 auseinander.

Zum Lösen der Teile einer Endoprothese wird die Vorrichtung 24 mit den Außenblättern 6 und dem Innenblatt 5 in der Ausgangsstellung in die Nut 4 der Endoprothese 13 eingeführt, wobei die halbkreisförmigen Ausnehmungen 29 der Schenkel 8 beziehungsweise der Außenblätter 6 und des Innenblattes 5 in die Nut um den Hals 2 der Endoprothese 13 eingreifen. In dieser Ausgangsposition ist das Innenblatt 5 gegenüber den Außenblättern 6 um ein bestimmtes Maß ausgefahren.
Anschließend wird der Schiebemechanismus durch entsprechende Drehung am Drehgriff 11 in Gang gesetzt, wodurch über die miteinander in Eingriff stehenden Gewinde 14 des Schiebeteils 20 und 17 der Welle 10 das Innenblatt 5 in Bezug auf Figur 1 nach links zwischen die Außenblätter 6 bis zur Enbeziehungsweise Lösestellung eingezogen wird In dieser Stellung sind die Außenflächen 21 der Außenblätter 6 im wesentlichen parallel zueinander angeordnet. Hierbei gleiten die korrespondierenden Schrägflächen 27, 28 aufeinander ab, wodurch die Drehbewegung des Drehgriffs 11 beziehungsweise der Welle 10 in eine Hubbewegung der Außenblätter 6 umgewandelt wird. Durch die äußerst geringe Neigung der Schrägflächen von bevorzugt ca. 2° und eine unter Umständen sehr geringe Steigung der Gewinde 14, 17 kann ein äußerst feinfühliges Auseinanderfahren der Außenblätter 6 gewährleistet werden.

Auch der für das Lösen der Teile der Endoprothese erforderliche Hub der Außenblätter 6 ist äußerst gering und liegt im Millimeterbereich, beispielsweise bei 1 mm oder sogar darunter. Aufgrund der im Vergleich zu diesem Hub sehr großen wirksamen Länge der schwenkbar gelagerten Schenkel 8 treten praktisch keine oder nur äußerst geringe Querkräfte auf, die von der Vorrichtung selbst jedoch abgefangen werden. Dadurch, daß zum Lösen der Teile der Endoprothese 13 praktisch ausschließlich Axialkräfte eingesetzt werden, die in Richtung der Halsachse 9 des Halses 2 der Endoprothese 13 wirken, wird ein äußerst schonendes Lösen der Endoprothesenteile gewährleistet. Durch den besonderen Verschiebemechanismus läßt sich auch eine gezielte Kraftdosierung beim Lösen der einzelnen Endoprothesenteile realisieren. In die gleiche Richtung wirkt auch der sehr klein gehaltene Konuswinkel 16, der Schrägflächen 27, 28 beziehungsweise der Außenblätter 6 beziehungsweise des Innenblattes 5, der eine leichte Kraftübertragung vom Innenblatt 5 auf die Außenblätter 6 ermöglicht. Insgesamt wird durch die erfindungsgemäße Vorrichtung ein sehr schonendes Lösen der Endoprothesenteile bewerkstelligt, wobei sowohl die Knochen als auch die Endoprothesenteile selbst nur einer erheblich reduzierten Belastung ausgesetzt sind, wobei insbesondere Querkräfte weitestgehend vermieden sind.

### Bezugszeichen

- 1: Gelenkkugel
- 2: Hals
- 3: Schaft
- 4: Nut
- 5: Innenblatt
- 6: Außenblatt
- 7: Bolzen
- 8: Schenkel
- 9: Halsachse
- 10: Welle
- 11: Drehgriff
- 12: Haltegriff
- 13: Endoprothese
- 14: Gewinde
- 15: Wellenlager
- 16: Konuswinkel
- 17: Gewinde
- 18: Verjüngung
- 19: Bolzenlager
- 20: Schiebeteil
- 21: Außenfläche
- 22: Axiallagerung
- 23: Verdickung
- 24: Vorrichtung
- 25: freies Ende
- 26: Innenfläche
- 27: Schrägfläche
- 28: Schrägfläche
- 29: Ausnehmung
- 30: Außenfläche
- 31: Längssteg
- 32: Ausnehmung

## Patentansprüche

1. Vorrichtung (24), insbesondere Abziehinstrument, zum Lösen der einzelnen Teile einer Endoprothese (13), beispielsweise für ein künstliches Hüftgelenk für Zwischenwirbel-Endoprothesen oder dergleichen, oder zum Spreizen von Wirbelsäulenkörpern, insbesondere zum Lösen des Preßsitzes von Schaft (3) und Hals (2) oder Hals (2) und Gelenkkugel (1) der Endoprothese (13), dadurch gekennzeichnet, daß die Vorrichtung (24) zwei Schenkel aufweist, deren freie Enden (25) in eine Nut (4) zwischen Schaft (3) und Hals (2) oder Hals (2) und Gelenkkugel (1) einführbar sind, und die Schenkel (8) mittels einer Spreizvorrichtung voneinander weg bewegbar sind.

2. Vorrichtung nach Anspruch 1, dadurch gekennzeichnet, daß die Schenkel (8) einends mittels eines Bolzenlagers (19), bevorzugt über Bolzen (7) an einem Haltegriff (12) schwenkbar gelagert sind.

3. Vorrichtung nach Anspruch 1 oder 2, dadurch gekennzeichnet, daß die freien Endabschnitte der Schenkel (8) anderenends jeweils als zungenartiges Außenblatt 6 ausgebildet sind.

4. Vorrichtung nach einem der vorhergehenden Ansprüche, dadurch gekennzeichnet, daß wenigstens eine, bevorzugt beide der einander zugewandten Innenflächen (26) der Schenkel (8) beziehungsweise Außenblätter (6) im Bereich des freien Endes (25) als Schrägflächen ausgebildet sind.

5. Vorrichtung nach Anspruch 4, dadurch gekennzeichnet, daß mittels der zum freien Ende (25) des Außenblattes (6) geneigte Schrägfläche eine Verjüngung (18) gebildet wird, wobei die Schrägfläche (27) bevorzugt einen Neigungsbeziehungsweise einen Konuswinkel von ca. 2° aufweist beziehungsweise bildet.

6. Vorrichtung nach einem der vorhergehenden Ansprüche, dadurch gekennzeichnet, daß die Spreizvorrichtung ein zwischen den Schenkeln (8) beziehungsweise Außenblättern (6) verschiebbar angeordnetes, zungenartiges Innenblatt (5) aufweist, welches im Bereich des freien Endes (25) einbevorzugt jedoch zweiseitig komplementäre Schrägflächen (28) besitzt, die jeweils bevorzugt eine Neigung beziehungsweise einen Konuswinkel (16) von ca. 2° aufweisen beziehungsweise bilden.

7. Vorrichtung nach Anspruch 6, dadurch gekennzeichnet, daß mittels der Schrägflächen (28) zum freien Ende des Innenblattes (5) eine Verdickung (23) gebildet ist.

8. Vorrichtung nach einem der vorhergehenden Ansprüche 3 bis 5, dadurch gekennzeichnet, daß die Außenblätter (6) sowie das Innenblatt (5) am freien Ende (25) eine bevorzugt mittig angeordnete, im wesentlichen bogenförmige ausgebildete Ausnehmung (29) besitzen, in der der Hals (2) der Endoprothese (13), im wesentlichen bündig dem Rand der Ausnehmung (13) anliegend, aufnehmbar ist.

9. Vorrichtung nach einem der vorhergehenden Ansprüche 3 bis 8, dadurch gekennzeichnet, daß die Außenflächen (21) der Außenblätter (6) in ihrer End- beziehungsweise Lösestellung, in der vorzugsweise die Innenflächen der Außenblätter (6) den Außenflächen (30) des Innenblattes (5) im wesentlichen bündig aufliegen, im wesentlichen parallel zueinander angeordnet.

10. Vorrichtung nach einem der vorhergehenden Ansprüche, dadurch gekennzeichnet, daß die Spreizvorrichtung einen Verschiebemechanismus für das Innenblatt (5) aufweist, mit dem das Innenblatt (5) weg von den freien Enden (20) der Schenkel (8) beziehungsweise Außenblätter (6) zwischen dieser einziehbar ist.

11. Vorrichtung nach Anspruch 10, dadurch gekennzeichnet, daß der Verschiebemechanismus durch eine Welle (10) mit Gewinde (17), eine Gewindespindel oder dergleichen gebildet ist, die drehbar in dem Haltegriff (12) aufgenommen ist und mit einem korrespondierenden Gewinde (14) eines endseitig des Innenblattes (5) angeordneten Schiebeteils (20) in Eingriff steht.

12. Vorrichtung nach Anspruch 11, dadurch gekennzeichnet, daß die Welle (10) in einem Wellenlager (15) am Haltegriff (12) gelagert, mittels einer Axiallagerung (22) gegen axiales Verschieben gesichert und bevorzugt am freien, aus dem Haltegriff (12) herausragenden Ende mit einem Drehgriff (11) verbunden ist.

13. Vorrichtung nach Anspruch 11 oder 12, dadurch gekennzeichnet, daß das Schiebeteil (20) einen oberen und/oder einen unteren Längssteg (31) besitzt, der oder die in einer korrespondierenden Schlitzung, Ausnehmung (32) oder dergleichen des oder der Schenkel (8) geführt sind.

14. Vorrichtung nach einem der vorhergehenden Ansprüche, dadurch gekennzeichnet, daß der Hub beziehungsweise die Spreizung der freien Enden der Schenkel (8) beziehungsweise Außenblätter (6) beim Einziehen des Innenblattes (5) zwischen die Außenblätter (6) unter 3%, bevorzugt unter 1%, besonders bevorzugt bei etwa 0,6 % der Schenkellänge zwischen freiem Ende (25) Schwenklager des Schenkels (8) liegt.

15. Vorrichtung nach Anspruch 13, dadurch gekennzeichnet, daß der maximale Hub unter ca. 1 mm und die Schenkellänge bei ca. 100 mm liegt.

16. Vorrichtung nach einem der vorhergehenden Ansprüche, dadurch gekennzeichnet, daß die Spreizvorrichtung die Schenkel (8) beziehungsweise Außenblätter (6) im wesentlichen axial, bevorzugt in Richtung der Halsachse (9) der Endoprothese (13) auseinanderbewegt.
